(19) Europäisches Patentamt
European Patent Office
Office européen des brevets

(11) Veröffentlichungsnummer: **0 226 988 B1**

(12) **EUROPÄISCHE PATENTSCHRIFT**

(45) Veröffentlichungstag der Patentschrift: **04.03.92**

(51) Int. Cl.$^5$: **C07C 45/50**, C07C 47/02

(21) Anmeldenummer: **86117394.6**

(22) Anmeldetag: **13.12.86**

# (54) Verfahren zur Herstellung von Aldehyden.

(30) Priorität: **24.12.85 DE 3546123**

(43) Veröffentlichungstag der Anmeldung:
**01.07.87 Patentblatt 87/27**

(45) Bekanntmachung des Hinweises auf die Patenterteilung:
**04.03.92 Patentblatt 92/10**

(84) Benannte Vertragsstaaten:
**AT BE CH DE ES FR GB IT LI LU NL SE**

(56) Entgegenhaltungen:
**EP-A- 0 159 460**
**EP-A- 0 163 234**
**FR-A- 2 314 910**
**FR-A- 2 478 078**
**FR-A- 2 489 308**

(73) Patentinhaber: **HOECHST AKTIENGESELLSCHAFT**
**Postfach 80 03 20**
**W-6230 Frankfurt am Main 80(DE)**

(72) Erfinder: **Cornils, Boy, Dr. Dipl.-Chem.**
**Kirschgartenstrasse 6**
**W-6238 Hofheim(DE)**
Erfinder: **Konkol, Werner, Dr. Dipl.-Chem.**
**Lützowstrasse 40 a**
**W-4200 Oberhausen 11(DE)**
Erfinder: **Bach, Hanswilhelm, Dr. Dipl.-Chem.**
**Alleestrasse 56**
**W-4100 Duisburg 11(DE)**
Erfinder: **Gick, Wilhelm, Dr. Dipl.-Chem.**
**Im Buschhuck 8**
**W4100 Duisburg 74(DE)**
Erfinder: **Wiebus, Ernst**
**Ferdinandstrasse 77**
**W-4200 Oberhausen 14(DE)**
Erfinder: **Bahrmann, Helmut, Dr. Dipl.-Chem.**
**Rohstrasse 48**
**W-4236 Hamminkeln-Brünen(DE)**
Erfinder: **Hahn, Heinz-Dieter, Dr. Dipl.-Chem.**
**Burgstrasse 21**
**W-4200 Oberhausen 11(DE)**

**EP 0 226 988 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von Aldehyden durch Hydroformylierung von Olefinen in Gegenwart wasserlöslicher Rhodium-Komplexverbindungen als Katalysator.

Es ist bekannt, durch Umsetzung von Olefinen mit Kohlenmonoxid und Wasserstoff Aldehyde und Alkohole herzustellen. Die Reaktion wird durch Hydridometallcarbonyle, vorzugsweise solcher der Metalle der 8. Gruppe des Periodensystems, katalysiert. Neben Kobalt, das als Katalysatormetall in großem Umfang technische Anwendung findet, gewinnt in letzter Zeit Rhodium zunehmende Bedeutung. Im Gegensatz zu Kobalt gestattet Rhodium, die Reaktion bei niedrigem Druck durchzuführen; darüber hinaus werden vorzugsweise geradkettige n-Aldehyde und nur in untergeordnetem Maße iso-Aldehyde gebildet. Schließlich ist auch die Hydrierung der Olefine zu gesättigten Kohlenwasserstoffen bei Anwendung von Rhodium-Katalysatoren deutlich niedriger als bei Anwendung von Kobalt-Katalysatoren.

Bei den in der Technik eingeführten Verfahren wird der Rhodium-Katalysator in Form von modifizierten Hydridorhodiumcarbonylen eingesetzt, die zusätzliche und gegebenenfalls überschüssige Liganden enthalten. Besonders bewährt haben sich als Liganden tertiäre organische Phosphine oder Ester der phosphorigen Säure. Ihre Anwendung ermöglicht es, den Reaktionsdruck auf Werte unter 30 MPa zu senken.

Probleme wirft bei diesem Verfahren jedoch die Abtrennung der Reaktionsprodukte und die Wiedergewinnung der im Reaktionsprodukt homogen gelösten Katalysatoren auf. Im allgemeinen destilliert man hierzu das Umsetzungsprodukt aus dem Reaktionsgemisch ab. In der Praxis kann dieser Weg wegen der thermischen Empfindlichkeit der gebildeten Aldehyde und Alkohole aber nur bei der Hydroformylierung niedriger Olefine, d.h. Olefine mit bis zu etwa 5 Kohlenstoffatomen im Molekül, beschritten werden. Außerdem hat sich gezeigt, daß die thermische Belastung des Destillationsgutes auch zu erheblichen Katalysatorverlusten durch Zersetzung der Rhodiumkomplexverbindungen führt.

Die geschilderten Mängel werden durch Anwendung von Katalysatorsystemen vermieden, die in Wasser löslich sind.

Derartige Katalysatoren sind z.B. in der DE-PS 26 27 354 beschrieben. Die Löslichkeit der Rhodiumkomplexverbindungen in Wasser wird durch Verwendung sulfonierter Triarylphosphine als Komplexligand erreicht. Die Abtrennung des Katalysators vom Reaktionsprodukt nach Beendigung der Hydroformylierungsreaktion erfolgt bei dieser Verfahrensvariante einfach durch Trennung von wäßriger und organischer Phase, d.h. ohne Destillation und damit ohne zusätzliche thermische Verfahrensschritte. Neben sulfonierten Triarylphosphinen werden als Komplexbestandteile wasserlöslicher Rhodiumkomplexverbindungen auch carboxylierte Triarylphosphine eingesetzt.

Die Reaktion des Olefins mit Kohlenmonoxid und Wasserstoff läuft in der wäßrigen, den Katalysator enthaltenden Phase ab. Die Rhodiumkonzentration in dieser Phase beträgt üblicherweise 50 bis 800 ppm, bezogen auf die wäßrige Lösung.

Ein Maß für die Wirksamkeit des aus Rhodium und wasserlöslichem Ligand bestehenden Katalysatorsystems ist die Anzahl der Mole Aldehyd, die je Volumeneinheit Katalysatorlösung und je Zeiteinheit gebildet werden. Für diese Beziehung wird im folgenden der Begriff "Produktivität" verwendet also

$$\text{Produktivität} = \frac{\text{Mole Aldehyd}}{\text{l Katalysatorlösung} \cdot \text{h}}$$

Mit steigender Rhodiummenge in der wäßrigen Katalysatorlösung nimmt die Produktivität zu. Darüber hinaus beeinflußt die Rhodiumkonzentration die Beständigkeit der sulfonierten oder carboxylierten Triarylphosphine. Mit steigender Rhodiumkonzentration nimmt die Neigung der Phosphor-Kohlenstoffbindung,unter Bildung z.B. von substituierten Phosphinsäurederivaten und Arylsulfonaten oder Arylcarboxylaten aufzuspalten, zu. Diese Reaktion hat schließlich zur Folge, daß die Aktivität des Katalysatorsystems zurückgeht.

Die Rhodium-Komplexverbindung enthält je Rhodiumatom maximal drei Phosphinmoleküle gebunden. Um ihre Stabilität zu erhöhen, empfiehlt es sich jedoch, bezogen auf vorhandenes Rhodium, einen hohen Phosphinüberschuß anzuwenden. Üblicherweise setzt man daher auf 1 g-Atom Rhodium 10 bis 100 Mol, bevorzugt 50 bis 100 Mol, wasserlösliches Phosphin ein. Ein hoher Phosphinüberschuß ist auch deshalb anzustreben, weil im Verlauf der Hydroformylierungsreaktion, insbesondere durch Hydrolyse und/oder Oxidation, irreversibel P(III)- und P(V)-verbindungen entstehen, die zur Komplexbildung mit Rhodium nicht mehr fähig sind.

Weiterhin fordert man von einem in der industriellen Praxis genutzten Katalysator, daß er auch bei voller Auslastung unter den für ihn charakteristischen Arbeitsbedingungen eine hohe Lebensdauer hat. Der

Zeitraum zwischen Neueinsatz des Katalysators und seinem Austausch wegen nicht mehr tolerierbarem Aktivitätsverlust gegen Frischkatalysator soll also möglichst lang sein.

Es bestand daher die Aufgabe ein Verfahren zur Herstellung von Aldehyden durch Umsetzung von aliphatischen Olefinen mit 2 bis 12 Kohlenstoffatomen mit Kohlenmonoxid und Wasserstoff in flüssiger Phase in Gegenwart von Wasser sowie von Rhodium in metallischer Form oder als Verbindung und dem wasserlöslichen Salz eines sulfonierten oder carboxylierten Triarylphosphins zu entwickeln, das hohe Produktivität der Katalysatorlösung mit langer Lebensdauer des Katalysatorsystems verbindet.

Diese Aufgabe wird bei einem Verfahren der obengenannten Art dadurch gelöst, daß die Rhodiumkonzentration in der Wasserphase 450 bis 800 Gew.-ppm und die Konzentration der sulfonierten oder carboxylierten Triarylphosphine 25 bis 30 Gew.-%, bezogen auf die wäßrige Lösung, beträgt.

Es hat sich gezeigt, daß die sorgfältige Abstimmung der Konzentration des Rhodiums und der Konzentration der sulfonierten oder carboxylierten Phosphine eine hohe Produktivität der Katalysatorlösung und eine lange Lebensdauer des Katalysators sicherstellt.

Auf den Einfluß steigender Rhodiumkonzentration auf die Katalysatorlebensdauer wurde bereits hingewiesen. Auch die Konzentration der sulfonierten oder carboxylierten Triarylphosphine in der wäßrigen Lösung wirkt auf die Lebensdauer des Katalysators ein. Steigende Phosphinkonzentration erhöht seine Lebensdauer.

Weiterhin wurde gefunden, daß die Produktivität des Katalysatorsystems außer von der Rhodiumkonzentration auch von der Konzentration der sulfonierten oder carboxylierten Triarylphosphine in der Katalysatorlösung abhängt. Eine Zunahme des Phosphinanteils in der Wasserphase führt zu einer Minderung der Geschwindigkeit der Hydroformylierungsreaktion und damit zu einem Rückgang der Produktivität des Katalysatorsystems.

Besonders bewährt hat es sich, die Konzentration der sulfonierten oder carboxylierten Triarylphosphine auf Werte von 26 bis 28 Gew.-%, bezogen auf die wäßrige Lösung, einzustellen.

Nach dem erfindungsgemäßen Verfahren lassen sich Olefine mit 2 bis 12 Kohlenstoffatomen hydroformylieren. Diese Olefine können linear oder verzweigt sein, mit einer endständigen oder innenständigen Doppelbindung. Beispiele für solche Olefine sind: Ethylen, Propylen, 1-Buten, 2-Buten, 1-Penten, 2-Methyl-1-buten, 4,4-Dimethyl-1-nonen, 1-Dodecen. Bevorzugt werden lineare Olefine mit 2 bis 8 Kohlenstoffatomen wie Ethylen, Propylen, 1-Buten, 1-Penten, 1-Hexen, 1-Hepten und 1-Octen eingesetzt.

Als Katalysator finden Rhodium oder Rhodiumverbindungen zusammen mit wasserlöslichen Phosphinen Anwendung, die der allgemeinen Formel

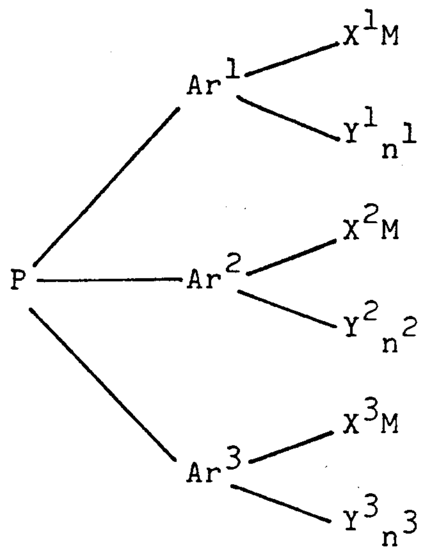

entsprechen. Hierbei bedeuten $Ar^1$, $Ar^2$, $Ar^3$ jeweils eine Phenyl- oder Naphthylgruppe, $Y^1$, $Y^2$, $Y^3$ jeweils eine geradkettige oder verzweigte Alkylgruppe mit 1 bis 4 C-Atomen, eine Alkoxygruppe, ein Halogenatom, die OH-, CN-, $NO_2$- oder $R^1R^2N$-Gruppen, in der $R^1$ und $R^2$ für jeweils eine geradkettige oder verzweigte Alkylgruppe mit 1 bis 4 C-Atomen stehen; $X^1$, $X^2$, $X^3$ ist jeweils ein Sulfonat-($SO_3^-$)Rest oder ein Carboxylat-($-COO^-$)-Rest, $n^1$, $n^2$, $n^3$ sind gleiche oder verschiedene ganze Zahlen von 0 bis 5, M ist ein Alkalimetallion, das Äquivalent eines Erdalkalimetall- oder Zinkions oder ein Ammonium- oder quartäres

Alkylammoniumion der allgemeinen Formel $N(R^3R^4R^5R^6)^+$, in der $R^3$, $R^4$, $R^5$, $R^6$ jeweils für eine geradkettige oder verzweigte Alkylgruppe insbesondere mit 1 bis 4 C-Atomen steht.

Nach einer bevorzugten Ausführungsform dieses Verfahrens werden als wasserlösliche Phosphine Verbindungen der vorstehend beschriebenen allgemeinen Formel eingesetzt, in der $Ar^1$, $Ar^2$, $Ar^3$ jeweils ein Phenylrest und $X^1$, $X^2$, $X^3$ jeweils einen Sulfonatrest oder einen Carboxylatrest bedeuten. Beispiele für Verbindungen der oben wiedergegebenen allgemeinen Formel sind Triphenylphosphin-tri-Natrisulfonat, Triphenylphosphin-tri-(tetraalkylammonium)-trisulfonat, Triphenylphosphin-tri-Na-tricarboxylat.

Die sulfonierten oder carboxylierten Arylphosphine können als einheitliche Verbindungen eingesetzt werden. Es können aber auch Gemische aus Phosphinen verwendet werden, die unterschiedlich viele Sulfonsäuregruppen oder Carboxylatgruppen enthalten, also z.B. Gemische aus Triarylphosphintrisulfonsäuren und Triarylphosphindisulfonsäuren. Darüber hinaus müssen die Sulfonate oder Carboxylate nicht dasselbe Kation enthalten. Auch Gemische aus Salzen, die sich von unterschiedlichen Metallen ableiten und/oder Ammonium- und/oder quartäre Alkylammoniumionen enthalten, sind geeignet.

Das Rhodium wird entweder in metallischer Form oder als Verbindung eingesetzt. Metallisches Rhodium wird bevorzugt auf einem Träger wie Aktivkohle, Calciumcarbonat, Aluminiumsilikat, Tonerde, aufgebracht verwendet. Als Rhodiumverbindungen kommen solche Substanzen in Betracht, die wasserlöslich sind oder unter den Reaktionsbedingungen wasserlöslich werden. Geeignete Verbindungen sind die verschiedenen Rhodiumoxide, Salze anorganischer Wasserstoff- und Sauerstoffsäuren, sowie Salze aliphatischer Mono- und Polycarbonsäuren. Als Beispiele seien genannt Rhodiumchlorid, Rhodiumnitrat, Rhodiumsulfat, Rhodiumacetat, Rhodiummalonat. Weiterhin können Rhodiumcarbonylverbindungen wie Tricarbonylrhodium oder Tetracarbonylrhodium oder Komplexsalze des Rhodiums, z.B. Cyclooctadienylrhodiumchlorid eingesetzt werden. Bevorzugt werden Rhodiumoxid, Rhodiumchlorid und Rhodiumacetat. Sowohl aus dem metallischen Rhodium als auch aus den Rhodiumverbindungen bilden sich unter den Reaktionsbedingungen die Kohlenmonoxid und Phosphine als Liganden enthaltenden Rhodium-Komplexverbindungen, die zusammen mit den überschüssigen Phosphinen das Katalysatorsystem bilden.

Die Katalysatorlösung kann im voraus, z.B. aus der wäßrigen Phosphinlösung und der benötigten Menge Rhodium, hergestellt und der Reaktionszone zugeführt werden. Es ist aber ebenso möglich, die Katalysatorlösung durch Vermischen der Komponenten in der Reaktionszone selbst herzustellen.

Das Rhodium liegt in der wäßrigen Katalysatorlösung in einer Konzentration von 450 bis 800 Gew.-ppm, vorzugsweise 500 bis 600 Gew.-ppm, bezogen auf die Lösung, vor.

Der Gesamtdruck von Wasserstoff und Kohlenmonoxid beträgt 1 bis 200 bar (100 bis $2 \cdot 10^4$ kPa), vorzugsweise 10 bis 100 bar ($1 \cdot 10^3$ bis $1 \cdot 10^4$ kPa). Die Zusammensetzung des Synthesegases, d.h. das Verhältnis von Kohlenmonoxid zu Wasserstoff kann in weiten Grenzen variiert werden. Im allgemeinen setzt man Synthesegas ein, in dem das Volumverhältnis von Kohlenmonoxid zu Wasserstoff 1 : 1 beträgt oder von diesem Wert nur wenig abweicht. Die Umsetzung erfolgt bei Temperaturen von 20 bis 150 °C, sie kann sowohl kontinuierlich als auch absatzweise durchgeführt werden.

Die nachfolgenden Beispiele erläutern die Erfindung.

Beispiel 1

In eine wäßrige Katalysatorlösung, die 27 Gew.-% eines Gemisches der Natriumsalze der Triphenylphosphintrisulfonsäure und der Triphenylphosphindisulfonsäure sowie 500 ppm Rhodium enthält, werden unter Rühren bei einer Temperatur von 122 °C, einem Druck von 50 MPa Propylen, Kohlenmonoxid und Wasserstoff im Vol.-Verhältnis 1 : 1 : 1 geleitet. Je 1 Katalysatorlösung und Stunde erhält man 1,95 Mole eines Gemisches aus n- und iso-Butyraldehyd.

Beispiel 2

Beispiel 1 wird wiederholt mit dem Unterschied, daß die Katalysatorlösung 30 Gew.-% eines Gemisches der Natriumsalze der Triphenylphosphintrisulfonsäure und der Triphenylphosphindisulfonsäure bei unveränderter Rhodiumkonzentration enthält. Je 1 Katalysatorlösung und Stunde werden 1,7 Mole eines Gemisches aus n- und iso-Butyraldehyd gebildet.

Beispiel 3 (zum Vergleich)

Beispiel 1 wird wiederholt mit dem Unterschied, daß die Katalysatorlösung 14,5 Gew.-% eines Gemisches der Natriumsalze der Triphenylphosphintrisulfonsäure und der Triphenyldisulfonsäure bei unveränderter Rhodium-Konzentration enthält. Je 1 Katalysatorlösung werden 3,1 Mol eines Gemisches aus n-

und iso-Butyraldehyd gebildet. Unter den genannten Reaktionsbedingungen wandeln sich die P(III)-verbindungen jedoch in erheblichem Umfang in P-(V)-verbindungen um, so daß die Katalysatorlösung schon nach kurzzeitigem Einsatz die ursprüngliche Aktivität einbüßt. Die gewählte Phosphinkonzentration ist daher für eine wirtschaftliche Anwendung der Arbeitsweise ungeeignet.

## Patentansprüche

1. Verfahren zur Herstellung von Aldehyden durch Umsetzung von aliphatischen Olefinen mit 2 bis 12 Kohlenstoffatomen mit Kohlenmonoxid und Wasserstoff in flüssiger Phase in Gegenwart von Wasser sowie von Rhodium in metallischer Form oder als Verbindung und dem wasserlöslichen Salz eines sulfonierten oder carboxylierten Triarylphosphins, dadurch gekennzeichnet, daß die Rhodium-Konzentration in der Wasserphase 450 bis 800 Gew.-ppm und die Konzentration der sulfonierten oder carboxylierten Triarylphosphine 25 bis 30 Gew.-%, jeweils bezogen auf die wäßrige Lösung, beträgt.

2. Verfahren nach Anspruch 1, dadurch gekennzeichnet, daß die Konzentration der sulfonierten oder carboxylierten Triarylphosphine in der wäßrigen Lösung 26 bis 28 Gew.-%, bezogen auf die wäßrige Lösung, beträgt.

3. Verfahren nach Anspruch 1 und 2, dadurch gekennzeichnet, daß die Rhodium-Konzentration in der wäßrigen Lösung 500 bis 600 Gew.-ppm, bezogen auf die wäßrige Lösung, beträgt.

## Claims

1. A process for the preparation of aldehydes by the reaction of aliphatic olefins with 2 to 12 carbon atoms with carbon monoxide and hydrogen in the liquid phase in the presence of water and rhodium in metallic form or as a compound and the water-soluble salt of a sulfonated or carboxylated triarylphosphine, characterised in that the rhodium concentration in the aqueous phase is 450 to 800 weight-ppm and the concentration of the sulfonated or carboxylated triarylphosphines is 25 to 30% by weight, in each case related to the aqueous solution.

2. A process according to claim 1, characterised in that the concentration of the sulfonated or carboxylated triarylphosphines in the aqueous solution is 26 to 28% by weight related to the aqueous solution.

3. A process according to claims 1 and 2, characterised in that the rhodium concentration in the aqueous solution is 500 to 600 weight-ppm, related to the aqueous solution.

## Revendications

1. Procédé pour la fabrication d'aldéhydes par réaction d'oléfines aliphatiques en $C_2$-$C_{12}$ avec le monoxyde de carbone et l'hydrogène en phase liquide en présence d'eau et de rhodium sous forme métallique ou sous forme de composé et du sel soluble dans l'eau d'une triarylphosphine sulfonée ou carboxylée, caractérisé en ce que la concentration de rhodium dans la phase aqueuse est de 450 à 800 ppm en poids et la concentration des triarylphosphines sulfonées ou carboxylées est de 25 à 30 % en poids, rapportées chaque fois à la solution aqueuse.

2. Procédé selon la revendication 1, caractérisé en ce que la concentration des triarylphosphines sulfonées ou carboxylées dans la solution aqueuse est de 26 à 28 % en poids, par rapport à la solution aqueuse.

3. Procédé selon la revendication 1 ou 2, caractérisé en ce que la concentration en rhodium dans la solution aqueuse est de 500 à 600 ppm en poids, par rapport à la solution aqueuse.